# EUROPÄISCHE PATENTANMELDUNG

(11) **EP 3 046 040 A1**
(43) Veröffentlichungstag der Anmeldung: **20.07.2016**
(21) Anmeldenummer: 15151603.6
(22) Anmeldetag: 19.01.2015
(51) Int. Cl.: G06F 19/00

(54) **Verfahren und Anordnung zum Ansteuern mindestens eines zum Durchführen zahnärztlicher Behandlungen und/oder Untersuchungen vorgesehenen Geräts**

(71) Anmelder: Kaltenbach & Voigt GmbH, 88400 Biberach (DE)
(72) Erfinder: Dicke, Torsten, 88400 Biberach (DE); Herrmann, Stefan, 88326 Aulendorf (DE)
(74) Vertreter: Thun, Clemens

(57) **Zusammenfassung**

Die Erfindung betrifft eine Anordnung bzw. ein Verfahren zum Ansteuern mindestens eines zum Durchführen zahnärztlicher Behandlungen und/oder Untersuchungen vorgesehenen Geräts (1), das die folgenden Schritte aufweist:
(a) Darstellen von Bedien- und/oder Steueroptionen für das Gerät (1) mit Hilfe einer graphischen Benutzeroberfläche (2) auf einer Anzeigeeinheit (3) eines Dental-Behandlungs- oder -Untersuchungsplatzes (4), wobei die Darstellung auf Basis von HTML-Dokumenten (101) erfolgt, welche der Anzeigeeinheit (3) von einem von dem Dental-Behandlungs- oder -Untersuchungsplatz (4) separaten Rechner (5) übermittelt werden, (b) Erzeugen von Steuerinformationen (102) durch Betätigen von Bedienoder Eingabeelementen (6) des Dental-Behandlungs- oder -Untersuchungsplatzes (4) und Übermittlung der Steuerinformationen (102) an den von dem Dental-Behandlungs- oder -Untersuchungsplatz (4) separaten Rechner (5) und (c) Umsetzen der Steuerinformationen (102) durch den Rechner (5) in für das Gerät (1) verständliche Steuerbefehle (103) und Übermitteln der Steuerbefehle (103) von dem Rechner (5) an das Gerät (1) zu dessen Ansteuerung. Ein Nutzer kann auf diese Weise mithilfe der Anzeigeeinheit (3) das Gerät (1) quasi fernsteuern. Er muss hierzu denjenigen Bereich, in dem er sich zur Durchführung seiner üblichen Arbeiten aufhält, nicht verlassen.

## Beschreibung

Die Erfindung betrifft ein Verfahren und eine Anordnung zum Ansteuern mindestens eines zum Durchführen zahnärztlicher Behandlungen und/oder Untersuchungen vorgesehenen Geräts.

Aus dem Stand der Technik ist ein zahnärztlicher Behandlungs- oder Untersuchungsplatz, hier auch als Dental-Behandlungs- oder -Untersuchungsplatz oder kurz als Behandlungsplatz bezeichnet bekannt. Der Behandlungsplatz umfasst einen Patientenstuhl für einen Patienten, sowie ein Bedienelement oder Arztelement, an dem Aufnahmen für dentale Handstücke ausgebildet sind, die zur Halterung entsprechender Handstücke dienen. Das Bedienelement weist außerdem eine Anzeigeeinheit auf, mit der beispielsweise Informationen über einen Betriebszustand eines der dentalen Handgeräte angezeigt und/oder eingestellt werden können.

Weiterhin ist bekannt, für einen solchen Behandlungslatz ein optionales Gerät vorzusehen, das zum Durchführen zahnärztlicher Behandlungen und/oder Untersuchungen vorgesehen ist; beispielsweise kann es sich bei dem optionalen Gerät um ein dentales Röntgengerät oder eine sog. Intraoralkamera handeln. Durch ein solches optionales Gerät lässt sich im Allgemeinen das Spektrum der Arbeiten, für die sich der Behandlungsplatz eignet, erweitern.

Wenn das optionale Gerät von einem Nutzer, also beispielsweise einer Zahnärztin, verwendet wird, muss es entsprechend bedient bzw. angesteuert werden. Dies ist üblicherweise mit einem besonderen Aufwand verbunden, der bereits durch die im Allgemeinen gegebene räumliche Trennung des optionalen Geräts von dem Bedienelement verursacht ist. Dabei entstehen durch eine Betätigung des Geräts durch den Nutzer im Allgemeinen auch zusätzliche Probleme hygienischer Art.

Der Erfindung liegt die Aufgabe zugrunde, ein verbessertes Verfahren bzw. eine verbesserte Anordnung zum Ansteuern mindestens eines zum Durchführen zahnärztlicher Behandlungen und/oder Untersuchungen vorgesehenen Geräts anzugeben. Insbesondere soll dabei eine erleichterte Bedienung des Geräts bzw. der Geräte ermöglicht sein.

Diese Aufgabe wird gemäß der Erfindung mit den in den unabhängigen Ansprüchen angegebenen Gegenständen gelöst. Bevorzugte Ausführungen der Erfindung sind in den abhängigen Ansprüchen angegeben.

Gemäß der Erfindung ist ein Verfahren zum Ansteuern mindestens eines zum Durchführen zahnärztlicher Behandlungen und/oder Untersuchungen vorgesehenen Geräts vorgesehen, das die folgenden Schritte aufweist:
a) Darstellen von Bedien- und/oder Steueroptionen für das Gerät mit Hilfe einer graphischen Benutzeroberfläche auf einer Anzeigeeinheit eines Dental-Behandlungs- oder -Untersuchungsplatzes, wobei die Darstellung auf Basis von HTML-Dokumenten (HTML: Hypertext Markup Language) erfolgt, welche der Anzeigeeinheit von einem von dem Dental-Behandlungs- oder -Untersuchungsplatz separaten Rechner übermittelt werden,
b) Erzeugen von Steuerinformationen durch Betätigen von Bedien- oder Eingabeelementen des Dental-Behandlungs- oder -Untersuchungsplatzes und Übermittlung der Steuerinformationen an den von dem Dental-Behandlungs- oder -Untersuchungsplatz separaten Rechner und
c) Umsetzen der Steuerinformationen durch den Rechner in für das Gerät verständliche Steuerbefehle und Übermitteln der Steuerbefehle von dem Rechner an das Gerät zu dessen Ansteuerung.

Ein Nutzer des Dental-Behandlungs- oder -Untersuchungsplatzes - hier im Folgenden auch kurz als Behandlungsplatz bezeichnet - kann auf diese Weise mithilfe der Anzeigeeinheit das Gerät sozusagen fernsteuern. Er muss hierzu also denjenigen Bereich, in dem er sich zur Durchführung seiner üblichen Arbeiten aufhält, nicht verlassen. Zudem lässt sich die Bedienung mit der ohnehin vorhandenen Anzeigeeinheit durchführen; somit ist es nicht erforderlich, dass ein entsprechend separates Eingabegerät zur Steuerung des Geräts zur Verfügung gestellt wird. Wenn das Gerät durch ein anderes entsprechendes Gerät ausgetauscht wird, muss die Anzeigeeinheit als solche nicht verändert werden. Ein entsprechender Vorteil ergibt sich auch, wenn das Gerät beispielsweise nachträglich, also im Sinn eines Nachrüstens in den Behandlungsplatz eingebunden wird oder wenn in analoger Weise ein entsprechendes weiteres Gerät in die Anordnung integriert wird.

Vorzugsweise ist vorgesehen, dass ein Aufruf bzw. eine Aktualisierung eines HTML-Dokuments sowohl durch den Rechner, als auch durch den Dental-Behandlungs- oder -Untersuchungsplatz initiiert werden kann. Hierdurch lässt sich insbesondere erzielen, dass eine Aktualisierung auch unabhängig von einer Betätigung der graphischen Benutzeroberfläche der Anzeigeeinheit oder einer anderweitigen Manipulation des Behandlungsplatzes stattfindet.

Vorzugsweise ist die Anzeigeeinheit des Dental-Behandlungs- oder -Untersuchungsplatzes berührungsempfindlich, wobei das Erzeugen der Steuerinformationen durch Berühren bzw. Betätigen der Anzeigeeinheit erfolgt. Dies ist besonders handhabungsfreundlich. Die Anzeigeeinheit lässt sich außerdem besonders gut reinigen, beispielsweise desinfizieren. Dies ist beispielsweise bei einer Tastatur oder einer Maus nicht der Fall.

Vorzugsweise erfolgt das Erzeugen der Steuerinformationen mit Hilfe eines Fußschalters des Dental-Behandlungs- oder -Untersuchungsplatzes. Auch dies ist besonders vorteilhaft mit Bezug auf die Bedienfreundlichkeit und die Einhaltung hygienischer Erfordernisse.

Vorzugsweise ist die Anzeigeeinheit des Dental-Behandlungs- oder -Untersuchungsplatzes ein so genannter Thin Client. Hierdurch lässt sich die Anzeigeeinheit besonders einfach und kleinräumig gestalten. Dies ist auch vorteilhaft mit Bezug auf die Gestaltungsmöglichkeiten des Behandlungsplatzes, insbesondere, wenn die Anzeigeeinheit als Teil eines Bedienelements des Behandlungsplatzes ausgebildet ist.

Vorzugsweise werden die HTML-Dokumente abhängig von der Identität einer zu behandelnden Person, von einem Betriebszustand des Dental-Behandlungs- oder -Untersuchungsplatzes, einer Eingabe an der Anzeigeeinheit und/oder einer sonstigen Betätigung des Dental-Behandlungs- oder -Untersuchungsplatzes, einschließlich der Betätigung eines angeschlossenen Fußschalters und/oder der Entnahme eines Dental-Behandlungs- oder -Untersuchungsgerätes dynamisch erzeugt. Hierdurch ist insbesondere eine weiterhin vereinfachte Handhabung und erhöhte Sicherheit gewährleistet.

Vorzugsweise handelt es sich bei dem anzusteuernden Gerät beispielsweise um ein Röntgengerät, eine intraorale Kamera, ein digitales Messsystem z.B. zur Karies-Diagnose, ein Patienten-Display und/oder eine Terminverwaltung. In diesen Fällen ist es besonders vorteilhaft, wenn eine Bedienung bzw. Ansteuerung des Geräts bzw. der Geräte bedienungsfreundlich und hygienisch über die graphische Benutzeroberfläche der Anzeigeeinheit erfolgen kann.

Vorzugsweise umfassen dabei die Steuerbefehle die Anzeige und/oder das Speichern von mit einem digitalen Device wie z. B. einer intraoralen Kamera aufgenommenen Daten wie z. B. Bildern und/oder Videos.

Vorzugsweise wird der Inhalt des angezeigten HTML-Dokuments aktualisiert, ohne das Dokument neu zu laden. Hierdurch lässt sich insbesondere auch erzielen, dass ein Benutzer ein Aktualisieren nicht aktiv auslösen muss.

Vorzugsweise erfolgt dabei die Aktualisierung allein durch einen mit der Anzeigeeinheit verbunden Server. Hierdurch lässt sich bei vergleichsweise einfacher Gestaltung eine hohe Sicherheit bzw. Zuverlässigkeit erzielen.

Vorzugsweise wird dabei das Übertragungsprotokoll für die aktualisierten Informationen vom Server an die Anzeigeeinheit automatisch ausgewählt, wobei vorzugsweise Web Sockets, Server Sent Events, Forever Frames oder Long Polling als Übertragungsprotokoll ausgewählt wird.

Gemäß einem weiteren Aspekt der Erfindung ist eine Anordnung zum Ansteuern mindestens eines zum Durchführen zahnärztlicher Behandlungen und/oder Untersuchungen vorgesehenen Geräts vorgesehen, die einen Dental-Behandlungs- oder -Untersuchungsplatz mit einer Anzeigeeinheit sowie einen separaten Rechner aufweist. Dabei ist der Dental-Behandlungs- oder -Untersuchungsplatz dazu ausgebildet, (i) auf der Anzeigeeinheit Bedien- und/oder Steueroptionen für das Gerät mit Hilfe einer graphischen Benutzeroberfläche dazustellen, wobei die Darstellung auf Basis von HTML-Dokumenten erfolgt, welche der Anzeigeeinheit von dem Rechner übermittelt werden, und (ii) bei Betätigen von Bedien- oder Eingabeelementen des Dental-Behandlungs- oder -Untersuchungsplatzes durch einen Benutzer Steuerinformationen zu erzeugen und diese an den Rechner zu übermitteln. Der Rechner ist dazu ausgebildet ist, die von dem Dental-Behandlungs- oder -Untersuchungsplatz empfangenen Steuerinformationen in für das Gerät verständliche Steuerbefehle umzusetzen und diese an das Gerät zu dessen Ansteuerung zu übermitteln.

Vorzugsweise ist dabei die Gestaltung derart, dass ein Aufruf bzw. eine Aktualisierung eines HTML-Dokuments sowohl durch den Rechner als auch durch den Dental-Behandlungs- oder -Untersuchungsplatz initiiert werden kann. Vorzugsweise ist die Anzeigeeinheit des Dental-Behandlungs- oder -Untersuchungsplatzes berührungsempfindlich, wobei die Gestaltung derart ist, dass das Erzeugen der Steuerinformationen durch Berühren bzw. Betätigen der Anzeigeeinheit erfolgt.

Vorzugsweise ist die Gestaltung weiterhin derart, dass das Erzeugen der Steuerinformationen mit Hilfe eines Fußschalters des Dental-Behandlungs- oder -Untersuchungsplatzes erfolgt. Vorzugsweise ist die Anzeigeeinheit des Dental-Behandlungs- oder -Untersuchungsplatzes ein Thin Client. Vorzugsweise ist die Gestaltung derart, dass die HTML-Dokumente abhängig von der Identität einer zu behandelnden Person, von einem Betriebszustand des Dental-Behandlungs- oder -Untersuchungsplatzes, einer Eingabe an der Anzeigeeinheit und/oder einer sonstigen Betätigung des Dental-Behandlungs- oder -Untersuchungsplatzes, einschließlich der Betätigung eines angeschlossenen Fußschalters und/oder der Entnahme eines Dental-Behandlungs- oder -Untersuchungsgerätes dynamisch erzeugt werden.

Vorzugsweise handelt es sich bei dem anzusteuernden Gerät beispielsweise um ein Röntgengerät, eine intraorale Kamera, ein digitales Messsystem z. B. zur Karies-Diagnose, ein Patienten-Display und/oder eine Terminverwaltung.

Vorzugsweise ist die Gestaltung derart, dass die Steuerbefehle die Anzeige und/oder das Speichern von mit einem digitalen Device wie z. B. einer intraoralen Kamera aufgenommenen Daten wie z. B. Bildern und/oder Videos umfassen.

Die Erfindung wird im Folgenden anhand eines Ausführungsbeispiels und mit Bezug auf die Zeichnung näher erläutert. Die Figur zeigt eine schematische Skizze zu einer erfindungsgemäßen Anordnung bzw. zu einem erfindungsgemäßen Verfahren.

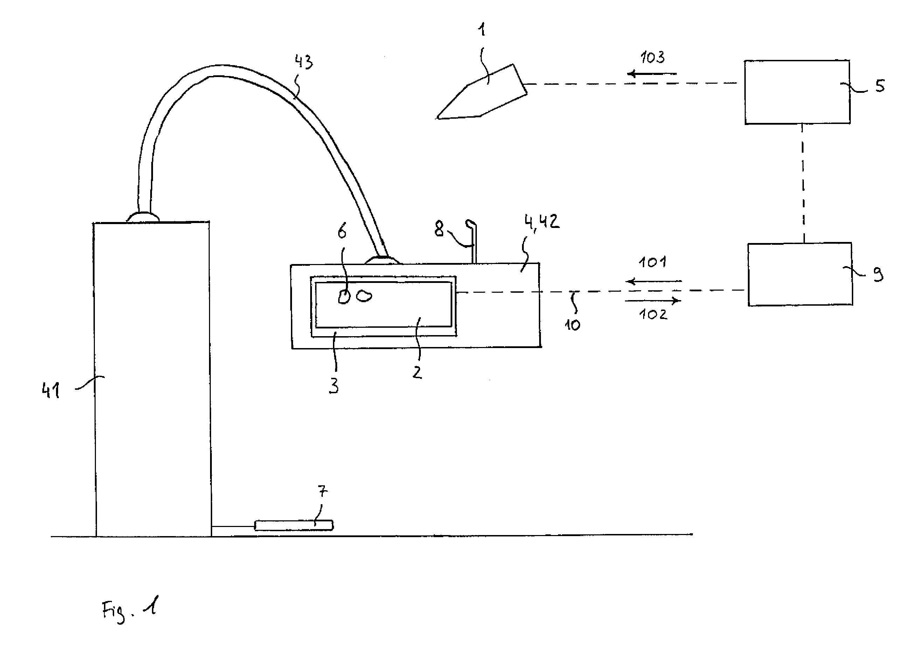

Wie in der Figur 1 sehr schematisch skizziert, umfasst eine erfindungsgemäße Anordnung einen zahnärztlichen Behandlungs- oder Untersuchungsplatz 4 bzw. einen Dental-Behandlungs- oder -Untersuchungsplatz 4, hier im Folgenden auch kurz als Behandlungsplatz 4 bezeichnet.

Der Behandlungsplatz 4 umfasst Vorrichtungen, die zur Durchführung zahnärztlicher Behandlungen und/oder Untersuchungen geeignet sind. Beispielsweise umfasst der Behandlungsplatz 4 als Basiselement ein säulenartiges Trägerelement 41, das dafür vorgesehen ist, auf eine Bodenfläche gestellt zu werden. An dem Trägerelement 41 ist vorzugsweise ein Bedienelement 42 angeordnet, beispielsweise schwenkbar gegenüber dem Trägerelement 41. Hierzu kann der Behandlungsplatz 4 einen Schwenkarm 43 aufweisen, der einerseits mit dem Trägerelement 41 und andererseits mit dem Bedienelement 42 verbunden ist. Der Schwenkarm 43 kann um eine beispielsweise vertikale Drehachse gegenüber dem Trägerelement 41 angeordnet sein; so lässt sich ein Schwenkbereich des Bedienelements 42 gegenüber dem Trägerelement 41 festlegen.

Weiterhin umfasst der Behandlungsplatz 4 vorzugsweise einen (in der Figur nicht gezeigten) Behandlungs- bzw. Patientenstuhl, der vorzugsweise dafür vorgesehen ist, ebenfalls auf die Bodenfläche gestellt zu werden und beispielsweise mit dem Trägerelement 41 verbunden sein kann. Weiterhin vorzugsweise umfasst der Behandlungsplatz 4 außerdem einen schematisch dargestellten Fußschalter 7, der beispielsweise mit dem Trägerelement 41 verbunden ist. Beispielsweise kann die Gestaltung derart sein, dass mithilfe des Fußschalters 7 eine Einstellung bzw. Positionierung des Behandlungsstuhls verändert werden kann. Auch die Ansteuerung von Behandlungsinstrumenten wie Bohrern oder dgl. über den Fußschalter ist aus dem Stand der Technik bekannt.

Weiterhin umfasst der Behandlungsplatz 4 vorzugsweise einen (in der Figur nicht gezeigten) Stuhl für einen Benutzer bzw. Nutzer der Anordnung, also beispielsweise für eine Zahnärztin.

An dem Bedienelement 42 ist vorzugsweise wenigstens eine beispielsweise köcherartige Halterung für wenigstens ein zahnärztliches Behandlungs- oder Untersuchungsgerät 8 bzw. ein Dental-Behandlungs- oder -Untersuchungsgerät 8, insbesondere in Form eines dentalen Handstücks ausgebildet. Bei dem wenigstens einen dentalen Handstück kann es sich beispielsweise um ein Turbinenhandstück oder um ein Handstück mit Elektroantrieb handeln. Außerdem kann das Bedienelement 42 eine nach oben weisende, als Instrumentenablage ausgebildete Ablagefläche aufweisen.

Weiterhin umfasst das Bedienelement 42 eine Anzeigeeinheit 3, auf der eine graphische Benutzeroberfläche 2 angezeigt werden kann. Grundsätzlich könnte die Anzeigeeinheit 3 jedoch auch in anderer Form an dem Behandlungsplatz 4 ausgebildet sein, beispielsweise als Teil des Trägerelements 41. Vorzugsweise ist die Gestaltung derart, dass mit der Anzeigeeinheit 3 Informationen über einen Betriebszustand des dentalen Handstücks angezeigt und/oder eingestellt werden können. Der Behandlungsplatz 4 ist so gestaltet, dass er sich ein- und ausschalten lässt, wobei durch ein Einschalten insbesondere auch die Anzeigeeinheit 3 eingeschaltet bzw. mit Strom versorgt wird.

Weiterhin umfasst die Anordnung ein zum Durchführen zahnärztlicher Behandlungen und/oder Untersuchungen vorgesehenes zusätzliches Gerät 1, beispielsweise in Form eines Röntgengeräts. Die Art, wie das Gerät 1 gehaltert ist, ist im hier betrachteten Rahmen grundsätzlich unerheblich. Beispielsweise kann das Gerät 1 an dem Trägerelement 41 gehaltert sein oder es kann eine hiervon separate Halterung für das Gerät 1 vorgesehen sein. Beispielsweise kann es sich bei dem Gerät 1 um ein Gerät handeln, das nicht dafür vorgesehen ist, an dem Bedienelement 42 gehaltert zu werden. Weiterhin umfasst die Anordnung einen Rechner 5, beispielsweise in Form eines PCs (PC: Personal Computer). Der Rechner 5 ist dabei von dem Behandlungsplatz 4 separiert; hiermit soll zum Ausdruck gebracht werden, dass der Rechner 5 beispielsweise ein Gehäuse aufweist, das nicht lagefest mit dem Behandlungsplatz 4 verbunden ist, insbesondere nicht an dem Behandlungsplatz 4 gehalten angeordnet ist. Beispielsweise kann vorgesehen sein, dass der Rechner 5 außerhalb eines Raumbereichs angeordnet ist bzw. wird, der durch die vertikale Projektion des Schwenkbereichs des Bedienelements 42 um das Trägerelement 41 festgelegt ist. So lässt sich zuverlässig verhindern, dass bei einem Schwenken des Bedienelements 5 Letzteres ungewollt gegen den Rechner 5 stößt.

Die Gestaltung des Behandlungsplatzes 4 ist derart, dass auf der Anzeigeeinheit 3 Bedien- und/oder Steueroptionen für das Gerät 1 mit Hilfe der graphischen Benutzeroberfläche 2 dargestellt werden können. Die Darstellung erfolgt dabei auf Basis von HTML-Dokumenten 101 (HTML: Hypertext Markup Language), die der Anzeigeeinheit 3 von dem Rechner 5 übermittelt werden.

Hierzu kann der Rechner 5 mit der Anzeigeeinheit 3 über ein Kabel 10 verbunden sein. Beispielsweise kann der Rechner 5 über ein LAN (local area network) mit der Anzeigeeinheit 4 verbunden sein; bei dem Kabel 10 kann es sich dementsprechend beispielsweise um ein LAN-Kabel handeln.

Beispielsweise kann die Gestaltung derart sein, dass zu einem Zeitpunkt bzw. innerhalb eines Zeitraums bzw. Zeitfensters eines der HTML-Dokumente 101 auf der Anzeigeeinheit 3 angezeigt werden kann. Vorzugsweise weist hierzu die Anzeigeeinheit 3 einen Web-Browser bzw. HTML-Browser zur Darstellung der HTML-Dokumente 101 auf.

Vorzugsweise ist der Rechner 5 mit einem Server 9, insbesondere in Form eines Web-Servers verbunden, mit dessen Hilfe die HTML-Dokumente 101 zur Verfügung gestellt werden bzw. auf der Anzeigeeinheit 3 angezeigt werden können. Der Server 9 ist dementsprechend mit der Anzeigeeinheit 3 verbunden.

Weiterhin ist der Behandlungsplatz 4 dazu ausgebildet, bei Betätigen von Bedien- oder Eingabeelementen 6 des Behandlungsplatzes 4 durch einen Nutzer Steuerinformationen 102 zu erzeugen und diese an den Rechner 5 zu übermitteln.

Der Rechner 5 ist dazu ausgebildet, die von dem Behandlungsplatz 4 empfangenen Steuerinformationen 102 in für das Gerät 1 verständliche Steuerbefehle 103 umzusetzen und diese an das Gerät 1 zu dessen Ansteuerung zu übermitteln. Hierzu ist der Rechner 5 vorzugsweise entsprechend mit dem Gerät 1 verbunden, beispielsweise über ein Kabel, beispielsweise in Form eines USB-Kabels (USB: Universal Serial Bus).

Beispielsweise ist die Gestaltung derart, dass die Bedien- oder Eingabeelemente 6 für den Benutzer erkennbar an dem Bedienelement 42 gebildet sind. Die Bedien- oder Eingabeelemente 6 können beispielsweise zumindest teilweise durch eine Darstellung auf der graphischen Benutzeroberfläche 2 der Anzeigeeinheit 3 gegeben bzw. gebildet sein. Insbesondere kann vorgesehen sein, dass die Bedien- oder Eingabeelemente 6 zumindest teilweise durch eine Darstellung auf einem der HTML-Dokumente 101, also sozusagen durch den Inhalt wenigstens eines der HTML-Dokumente 101 gegeben sind. Hierdurch lässt sich erzielen, dass sich auf der Anzeigeeinheit 3 zeitlich nacheinander unterschiedliche der Bedien- oder Eingabeelemente 6 anzeigen lassen oder es kann vorgesehen sein, dass zumindest ein Teil der Bedien- oder Eingabeelemente 6 innerhalb eines ersten Zeitfensters auf der Anzeigeeinheit 3 dargestellt werden, jedoch innerhalb eines zweiten Zeitfensters, das nicht mit dem ersten Zeitfenster überlappt, keines der Bedien- oder Eingabeelemente 6 auf der Anzeigeeinheit 3 angezeigt bzw. dargestellt wird. Dies ist beispielsweise vorteilhaft, wenn der Nutzer innerhalb des ersten Zeitfensters das Gerät 1 ansteuern will, während des zweiten Zeitfensters jedoch nicht, also beispielsweise während des zweiten Zeitfensters einen Betriebszustand des dentalen Handstücks 8 mit Hilfe der Anzeigeeinheit 3 einstellen will. Auch wäre denkbar, dass an den Rechner 5 mehrere zusätzliche Geräte angeschlossen sind und zu einem bestimmten Zeitpunkt immer nur die Bedienoptionen bezüglich eines der Geräte dargestellt werden.

Grundsätzlich ist hierdurch also ermöglicht, dass mithilfe der Anzeigeeinheit 3 sowohl eine Einstellung oder Ansteuerung des wenigstens einen dentalen Handstücks 8 vorgenommen werden kann, als auch das zusätzliche Gerät 1 angesteuert werden kann. Ein Nutzer der Anordnung kann so das Gerät 1 sozusagen fernsteuern, ohne seinen Platz unmittelbar neben dem Bedienelement 42 hierfür verlassen zu müssen.

Dabei besteht ein besonderer Vorteil der Erfindung darin, dass die Bedienoptionen leidglich in Form der bereits erwähnten HTML-Dokumente zur Verfügung gestellt werden. Dies bedeutet, dass an die Intelligenz der Anzeigeeinheit 3 nur geringe Anforderungen gestellt werden und das Gesamtsystem sehr flexibel die Hinzufügung zusätzlicher neuer Geräte ermöglicht. Es ist dann lediglich erforderlich, dass der PC zu diesem neuen Gerät gehörende HTML-Seiten zur Verfügung stellt, an der Software der Anzeigeeinheit 3 selbst müssen hingegen keine Änderungen vorgenommen werden.

Vorzugsweise ist die Anzeigeeinheit 3 berührungsempfindlich; beispielsweise kann sie hierzu einen Touchscreen bzw. einen Touch-Panel zur Darstellung der graphischen Benutzeroberfläche 2 aufweisen, beispielsweise einen kapazitiven Touchscreen. Dabei ist die Gestaltung weiterhin vorzugsweise derart, dass das Erzeugen der Steuerinformationen 102 durch ein Berühren bzw. Betätigen der Anzeigeeinheit 3 erfolgt, also beispielsweise durch ein Berühren bzw. Betätigen der auf dem Touchscreen angezeigten graphischen Benutzeroberfläche 2.

Die Gestaltung kann vorzugsweise auch alternativ oder zusätzlich hierzu derart sein, dass das Erzeugen der Steuerinformationen 102 mit Hilfe des Fußschalters 7 des Behandlungsplatzes 4 erfolgt. In diesem Fall sind dann wiederum auf dem Display zumindest Informationen dargestellt, die Auskunft darüber geben, im Hilfe welcher Betätigungen des Fußschalters 7 eine Ansteuerung des Geräts 1 vorgenommen werden kann.

Vorzugsweise ist die Anzeigeeinheit 3 als Thin Client ausgebildet, derart, dass die Anzeigeeinheit 3 zur Erzeugung der graphischen Benutzeroberfläche 2 abhängig von dem Rechner 5 und/oder dem Server 9 gestaltet ist. Mit anderen Worten ist die Anzeigeeinheit 3 als embedded Computer ausgebildet. Dabei kann eine Kommunikation mit dem Rechner 5 über Standard-Bibliotheken in JAVA-Skript erfolgen. Auf diese Weise lässt sich eine Darstellung der HTML-Dokumente 101 auf der Anzeigeeinheit 3 bewirken, die grundsätzlich von der übrigen Hardware und Software der Anordnung unabhängig ist.

Außerdem lässt sich hierdurch die Anzeigeeinheit 3 prinzipiell besonders einfach und kleinräumig gestalten. Dies ist insbesondere vorteilhaft mit Bezug auf die Gestaltungsmöglichkeiten des Bedienelements 42.

Zum Ansteuern des Geräts 1 sind erfindungsgemäß folgende Schritte vorgesehen:
(a) Darstellen der Bedien- und/oder Steueroptionen für das Gerät 1 mit Hilfe der graphischen Benutzeroberfläche 2 auf der Anzeigeeinheit 3 des Behandlungsplatzes 4, wobei die Darstellung auf Basis der HTML-Dokumente 101 erfolgt, welche der Anzeigeeinheit 3 von dem Rechner 5 übermittelt werden,
(b) Erzeugen der Steuerinformationen 102 durch das Betätigen der Bedien- oder Eingabeelemente 6 des Behandlungsplatzes 4 und Übermittlung der Steuerinformationen 102 an den Rechner 5,
(c) Umsetzen der Steuerinformationen 102 durch den Rechner 5 in die für das Gerät 1 verständlichen Steuerbefehle 103 und Übermitteln der Steuerbefehle 103 von dem Rechner 5 an das Gerät 1 zu dessen Ansteuerung.

Auf diese Weise ist es für den Nutzer insbesondere möglich, das Gerät 1 lediglich durch eine Berührung des Behandlungsplatzes 4 anzusteuern. Dies ist vorteilhaft, weil durch eine Berührung eines anderweitigen Elements, also beispielsweise einer an dem Gerät 1 ausgebildeten Schaltfläche, ein zusätzliches hygienisches Problem verursacht wäre. Die Einhaltung hygienischer Sicherheitsstandards hat insbesondere im Rahmen dentaler Arbeiten naturgemäß einen besonders hohen Stellenwert.

Wie oben angegeben, kann es sich bei dem zum Durchführen zahnärztlicher Behandlungen und/oder Untersuchungen vorgesehenen Gerät 1 beispielsweise um ein Röntgengerät handeln. Weiterhin kann es sich bei dem Gerät 1 beispielsweise um eine intraorale Kamera handeln oder um ein digitales Messsystem, zum Beispiel zur Karies-Diagnose oder ein Patienten-Display oder eine Terminverwaltung bzw. eine Hardwarekomponente zum Ausführen einer Praxis Management Software zur Verwaltung von Terminen.

Insbesondere kann vorgesehen sein, dass neben dem genannten Gerät 1 wenigstens ein weiteres, entsprechendes Gerät (in der Figur nicht gezeigt) mit dem Rechner 5 verbunden ist und in analoger Weise angesteuert werden kann.

Die genannte Gestaltung ist wie bereits angedeutet besonders vorteilhaft, weil bei einer Veränderung der Konfiguration der entsprechenden Geräte der Anordnung eine erforderliche Anpassung der Ansteuerung für den Nutzer besonders einfach möglich ist. Beispielsweise kann hierbei die Gestaltung weiterhin derart sein, dass sich das wenigstens eine weitere Gerät mittels Plug-in mit dem Rechner 5 verbinden und so in die Anordnung integrieren lässt.

Dabei ist die Gestaltung weiterhin vorzugsweise derart, dass die Steuerbefehle 103 eine Anzeige und/oder ein Speichern von mit einem digitalen Device, beispielsweise der intraoralen Kamera aufgenommenen Daten umfassen. Bei den Daten kann es sich also insbesondere um Daten zur Darstellung von Bildern und/oder Videos handeln. Dies ist beispielsweise vorteilhaft, wenn es sich bei dem Gerät 1 um ein Patienten-Display handelt und ein weiteres entsprechendes Gerät durch die intraorale Kamera gegeben ist. Hier lassen sich mit der intraoralen Kamera Bilder erzeugen und auf dem Patienten-Display darstellen, wobei sowohl die intraorale Kamera, als auch das Patienten-Display von dem Nutzer mithilfe der Anzeigeeinheit 3, möglicherweise in Verbindung mit dem Fußschalter 7 angesteuert werden können.

Wie erwähnt, erfolgt die Darstellung der Bedien- und/oder Steueroptionen für das Gerät 1 auf der Anzeigeeinheit 3 mit Hilfe der graphischen Benutzeroberfläche 2, wobei diese Darstellung auf Basis der HTML-Dokumente 101 erfolgt, die der Anzeigeeinheit 3 von dem Rechner 5 übermittelt werden. Im gezeigten Beispiel umfasst die Anordnung hierzu den Server 9 in Form eines Web-Servers, mit dessen Hilfe die HTML-Dokumente 101 zur Verfügung gestellt werden.

Vorzugsweise ist die Gestaltung der Anordnung derart, dass die HTML-Dokumente 101 dynamisch erzeugt werden. Dabei erfolgt die dynamische Erzeugung vorzugsweise in Abhängigkeit einer Identität einer, mit dem Behandlungsplatz 4 zu behandelnden Person und/oder in Abhängigkeit von einem Betriebszustand des Behandlungsplatzes 4 und/oder in Abhängigkeit einer Eingabe an der Anzeigeeinheit 3 und/oder einer sonstigen Betätigung des Behandlungsplatzes 4, einschließlich einer Betätigung des Fußschalters 7 und/oder in Abhängigkeit einer Entnahme des Dental-Behandlungs- oder -Untersuchungsgeräts 8, beispielsweise aus der entsprechenden an dem Bedienelement 42 ausgebildeten Halterung.

Wie weiter oben erwähnt, kann vorgesehen sein, dass das Gerät 1 nicht dafür vorgesehen ist, an dem Bedienelement 42 gehaltert zu werden. Allerdings kann grundsätzlich vorteilhaft beispielsweise vorgesehen sein, dass mehrere entsprechende Geräte mit Hilfe der Anzeigeeinheit 3 ansteuerbar sind, wobei wenigstens eines dieser Geräte - beispielsweise die oben beispielhaft genannte intraorale Kamera - in ihrer Ruhe- bzw. Nichtgebrauchsposition an dem Bedienelement 42 gehaltert ist.

Vorzugsweise ist die graphische Benutzeroberfläche 2 in einer SQL-Datenbank des Servers 9 hinterlegt und wird bei Benutzung dynamisch geladen.

Vorzugsweise ist die Gestaltung der Anordnung derart, dass ein Aufruf eines der HTML-Dokumente 101 sowohl durch den Rechner 5, als auch durch den Behandlungsplatz 4 initiiert werden kann. Außerdem ist die Gestaltung vorzugsweise derart, dass eine Aktualisierung desjenigen der HTML-Dokumente 101, das gerade auf der Anzeigeeinheit 3 dargestellt wird, sowohl durch den Rechner 5, als auch durch den Behandlungsplatz 4 initiiert werden kann.

Hierdurch lässt sich erzielen, dass die Aktualisierung durchgeführt wird, ohne dass hierzu eine Aktion des Nutzers erforderlich ist, also insbesondere ohne dass der Nutzer den Behandlungsplatz 4, also beispielsweise die Bedieneinheit 42 berührt.

Insbesondere kann dabei vorgesehen sein, dass innerhalb eines Zeitraums, in dem die Darstellung der Bedien- und/oder Steueroptionen für das Gerät 1 auf Basis eines der HTML-Dokumente 101 erfolgt, eine Aktualisierung des betreffenden HTML-Dokuments sowohl durch den Rechner 5, als auch durch den Behandlungsplatz 4 initiiert werden kann. Hierbei kann insbesondere vorgesehen sein, dass die Aktualisierung erfolgt, indem lediglich bestimmte Inhalte des betreffenden HTML-Dokuments verändert bzw. aktualisiert werden, wobei das betreffende HTML-Dokument als solches nicht neu auf die Anzeigeeinheit 3 bzw. den HTML-Browser der Anzeigeeinheit 3 geladen wird - also ohne ein Page-Reload.

Vorzugsweise erfolgt dabei die Aktualisierung allein durch den Sever 9, der hierzu mit der Anzeigeeinheit 3 entsprechend verbunden ist.

Weiterhin vorzugsweise ist die Gestaltung dabei derart, dass ein Übertragungsprotokoll für die aktualisierten Informationen bzw. den aktualisierten Inhalt des betreffenden HTML-Dokuments vom Server 9 an die Anzeigeeinheit 3 automatisch ausgewählt wird. Vorzugsweise wird dabei eines der vier folgenden Übertragungsprotokolle ausgewählt: Web Sockets, Server Sent Events, Forever Frames oder Long Polling, insbesondere in dieser Rangfolge.

Eine Zuordnung zwischen dem Rechner 5 und der Anzeigeeinheit 3 erfolgt vorzugsweise dynamisch nach einem Einschalten der Behandlungseinheit 4 bzw. der Anzeigeeinheit 3.

Wenn die Anordnung außer dem Gerät 1 in analoger Weise noch wenigstens ein weiteres Gerät aufweist, lässt sich erzielen, dass jedes dieser Geräte mithilfe der Anzeigeeinheit 3 ferngesteuert werden kann. Dabei ist vorzugsweise vorgesehen, dass beim Einschalten der Behandlungseinheit 4 alle entsprechenden Geräte mit dem Rechner 5 und der Anzeigeeinheit 3 entsprechend verknüpft werden. Dies ist besonders vorteilhaft, wenn sich die Konfiguration der in die Anordnung integrierten Geräte verändert. Vorzugsweise ist die Gestaltung dabei derart, dass die "Infrastruktur" der Geräte mithilfe der Anzeigeeinheit 3 konfiguriert werden kann, beispielsweise mit Hilfe eines Floor Plan. Auch ist hierbei ein Nachrüsten der Behandlungseinheit 4 mit einem entsprechenden weiteren Gerät besonders handhabungsfreundlich ermöglicht.

Grundsätzlich kann anstelle des Touchscreens bzw. der Anzeigeeinheit 3 auch ein anderes Eingabegerät vorgesehen sein, beispielsweise ein Tablet, insbesondere ein mobiles Tablet oder ein Smartphone.

## Patentansprüche

1. Verfahren zum Ansteuern mindestens eines zum Durchführen zahnärztlicher Behandlungen und/oder Untersuchungen vorgesehenen Geräts (1), wobei das Verfahren aufweist:
a) Darstellen von Bedien- und/oder Steueroptionen für das Gerät (1) mit Hilfe einer graphischen Benutzeroberfläche (2) auf einer Anzeigeeinheit (3) eines Dental-Behandlungs- oder -Untersuchungsplatzes (4), wobei die Darstellung auf Basis von HTML-Dokumenten (101) erfolgt, welche der Anzeigeeinheit (3) von einem von dem Dental-Behandlungs- oder -Untersuchungsplatz (4) separaten Rechner (5) übermittelt werden,
b) Erzeugen von Steuerinformationen (102) durch Betätigen von Bedien- oder Eingabeelementen (6) des Dental-Behandlungs- oder -Untersuchungsplatzes (4) und Übermittlung der Steuerinformationen (102) an den von dem Dental-Behandlungs- oder -Untersuchungsplatz (4) separaten Rechner (5),
c) Umsetzen der Steuerinformationen (102) durch den Rechner (5) in für das Gerät (1) verständliche Steuerbefehle (103) und Übermitteln der Steuerbefehle (103) von dem Rechner (5) an das Gerät (1) zu dessen Ansteuerung.

2. Verfahren nach Anspruch 1,
**dadurch gekennzeichnet,**
**dass** ein Aufruf bzw. eine Aktualisierung eines HTML-Dokuments sowohl durch den Rechner (5) als auch durch den Dental-Behandlungs- oder -Untersuchungsplatz (4) initiiert werden kann.

3. Verfahren nach Anspruch 1 oder 2,
**dadurch gekennzeichnet,**
**dass** die Anzeigeeinheit (3) des Dental-Behandlungs- oder -Untersuchungsplatzes (4) berührungsempfindlich ist und das Erzeugen der Steuerinformationen (102) durch Berühren bzw. Betätigen der Anzeigeeinheit (3) erfolgt.

4. Verfahren nach einem der vorherigen Ansprüche,
**dadurch gekennzeichnet,**
**dass** das Erzeugen der Steuerinformationen (102) mit Hilfe eines Fußschalters (7) des Dental-Behandlungs- oder -Untersuchungsplatzes (4) erfolgt.

5. Verfahren nach einem der Ansprüche 1 bis 4,
**dadurch gekennzeichnet,**
**dass** die Anzeigeeinheit (3) des Dental-Behandlungs- oder -Untersuchungsplatzes (4) ein Thin Client ist.

6. Verfahren nach einem der vorherigen Ansprüche,
**dadurch gekennzeichnet,**
**dass** die HTML-Dokumente (101) abhängig von der Identität einer zu behandelnden Person, vom Betriebszustand des Dental-Behandlungs- oder -Untersuchungsplatzes (4), einer Eingabe an der Anzeigeeinheit (3) und/oder einer sonstigen Betätigung des Dental-Behandlungs- oder -Untersuchungsplatzes (4), einschließlich der Betätigung eines angeschlossenen Fußschalters (7) und/oder der Entnahme eines Dental-Behandlungs- oder -Untersuchungsgerätes (8) dynamisch erzeugt werden.

7. Verfahren nach einem der vorherigen Ansprüche,
**dadurch gekennzeichnet,**
**dass** es sich bei dem anzusteuernden Gerät (1) beispielsweise um ein Röntgengerät, eine intraorale Kamera, ein digitales Messsystem z. B. zur Karies-Diagnose, ein Patienten-Display und/oder eine Terminverwaltung handelt.

8. Verfahren nach Anspruch 7,
**dadurch gekennzeichnet,**
**dass** die Steuerbefehle (103) die Anzeige und/oder das Speichern von mit einem digitalen Device wie z. B. einer intraoralen Kamera aufgenommenen Daten wie z. B. Bildern/Videos umfassen.

9. Verfahren nach einem der vorherigen Ansprüche,
**dadurch gekennzeichnet,**
**dass** der Inhalt des angezeigten HTML-Dokuments aktualisiert wird, ohne das Dokument neu zu laden.

10. Verfahren nach Anspruch 9,
**dadurch gekennzeichnet,**
**dass** die Aktualisierung allein durch einen mit der Anzeigeeinheit (3) verbunden Server (9) erfolgt.

11. Verfahren nach Anspruch 10,
**dadurch gekennzeichnet,**
**dass** das Übertragungsprotokoll für die aktualisierten Informationen vom Server (9) an die Anzeigeeinheit (3) automatisch ausgewählt wird, wobei vorzugsweise Web Sockets, Server Sent Events, Forever Frames oder Long Polling als Übertragungsprotokoll ausgewählt wird.

12. Anordnung zum Ansteuern mindestens eines zum Durchführen zahnärztlicher Behandlungen und/oder Untersuchungen vorgesehenen Geräts (1), aufweisend einen Dental-Behandlungs- oder -Untersuchungsplatz (4) mit einer Anzeigeeinheit (3) sowie einen separaten Rechner (5), wobei
a) der Dental-Behandlungs- oder -Untersuchungsplatz (4) dazu ausgebildet ist,
i) auf der Anzeigeeinheit (3) Bedien- und/oder Steueroptionen für das Gerät (1) mit Hilfe einer graphischen Benutzeroberfläche (2) dazustellen, wobei die Darstellung auf Basis von HTML-Dokumenten (101) erfolgt, welche der Anzeigeeinheit (3) von dem Rechner (5) übermittelt werden, und
ii) bei Betätigen von Bedien- oder Eingabeelementen (6) des Dental-Behandlungs- oder -Untersuchungsplatzes (4) durch einen Benutzer Steuerinformationen (102) zu erzeugen und diese an den Rechner (5) zu übermitteln,
b) der Rechner (5) dazu ausgebildet ist, die von dem Dental-Behandlungs- oder
- Untersuchungsplatz (4) empfangenen Steuerinformationen (102) in für das Gerät (1) verständliche Steuerbefehle (103) umzusetzen und diese an das Gerät (1) zu dessen Ansteuerung zu übermitteln.

13. Anordnung nach Anspruch 12,
**dadurch gekennzeichnet,**
**dass** ein Aufruf bzw. eine Aktualisierung eines HTML-Dokuments sowohl durch den Rechner (5) als auch durch den Dental-Behandlungs- oder -Untersuchungsplatz (4) initiiert werden kann.

14. Anordnung nach Anspruch 12 oder 13,
**dadurch gekennzeichnet,**
**dass** die Anzeigeeinheit (3) des Dental-Behandlungs- oder -Untersuchungsplatzes (4) berührungsempfindlich ist und das Erzeugen der Steuerinformationen (102) durch Berühren bzw. Betätigen der Anzeigeeinheit (3) erfolgt.

15. Anordnung nach einem der Ansprüche 12 bis 14,
**dadurch gekennzeichnet,**
**dass** das Erzeugen der Steuerinformationen (102) mit Hilfe eines Fußschalters (7) des Dental-Behandlungs- oder -Untersuchungsplatzes (4) erfolgt.

16. Anordnung nach einem der Ansprüche 12 bis 15,
**dadurch gekennzeichnet,**
**dass** die Anzeigeeinheit (3) des Dental-Behandlungs- oder -Untersuchungsplatzes (4) ein Thin Client ist.

17. Anordnung nach einem der Ansprüche 12 bis 16,
**dadurch gekennzeichnet,**
**dass** die HTML-Dokumente (101) abhängig von der Identität einer zu behandelnden Person, vom Betriebszustand des Dental-Behandlungs- oder -Untersuchungsplatzes (4), einer Eingabe an der Anzeigeeinheit (3) und/oder einer sonstigen Betätigung des Dental-Behandlungs- oder -Untersuchungsplatzes (4), einschließlich der Betätigung eines angeschlossenen Fußschalters (7) und/oder der Entnahme eines Dental-Behandlungs- oder -Untersuchungsgerätes (8) dynamisch erzeugt werden.

18. Anordnung nach einem der Ansprüche 12 bis 17,
**dadurch gekennzeichnet,**
**dass** es sich bei dem anzusteuernden Gerät (1) beispielsweise um ein Röntgengerät, eine intraorale Kamera, ein digitales Messsystem z. B. zur Karies-Diagnose, ein Patienten-Display und/oder eine Terminverwaltung handelt.

19. Anordnung nach Anspruch 18,
**dadurch gekennzeichnet,**
**dass** die Steuerbefehle (103) die Anzeige und/oder das Speichern von mit einem digitalen Device wie z. B. einer intraoralen Kamera aufgenommenen Daten wie z. B. Bildern/Videos umfassen.
